# FASCICULE DE BREVET EUROPEEN

(11) **EP 3 389 777 B1**
(45) Date de publication et mention de la délivrance du brevet: **04.12.2019**
(21) Numéro de dépôt: 16813101.9
(22) Date de dépôt: 07.12.2016
(51) Int. Cl.: A61N 5/06

(54) **DISPOSITIF POUR LA STIMULATION OPTIQUE DU CERVEAU AU MOYEN D'UNE FIBRE OPTIQUE**
VORRICHTUNG ZUR OPTISCHEN STIMULATION DES GEHIRNS ÜBER EINE OPTISCHE FASER
DEVICE FOR OPTICALLY STIMULATING THE BRAIN VIA AN OPTICAL FIBER

(30) Priorité: 16.12.2015 FR 1562525
(43) Date de publication de la demande: 24.10.2018
(73) Titulaire: Commissariat à l'Energie Atomique et aux Energies Alternatives, 75015 Paris (FR)
(72) Inventeur: CHABROL, Claude, 38320 Poisat (FR)
(74) Mandataire: Cabinet Beaumont
(86) Numéro de dépôt international: PCT/FR2016/053249
(87) Numéro de publication internationale: WO 2017/103381

(56) Documents cités:
- EP-A2- 0 206 943
- FR-A1- 3 010 321
- US-A1- 2004 015 211
- US-A1- 2011 125 077

## Description

### Domaine

La présente demande concerne le domaine des dispositifs optiques de façon générale, et vise en particulier un dispositif optique implantable pour la stimulation profonde du cerveau humain ou animal par irradiation optique.

### Exposé de l'art antérieur

La stimulation cérébrale profonde ("deep brain stimulation" en anglais) est une technique thérapeutique comportant l'implantation dans le cerveau du patient d'un dispositif permettant de stimuler des parties spécifiques du cerveau. On a notamment proposé des traitements de certains dysfonctionnements neuronaux, dont la maladie de parkinson, par irradiation optique de parties du cerveau avec une source de lumière émettant dans le domaine de l'infrarouge.

On a déjà proposé des dispositifs implantables permettant de mettre en oeuvre des traitements par stimulation optique profonde du cerveau au moyen d'une fibre optique introduite dans le cerveau du patient, par l'intermédiaire de laquelle de la lumière provenant d'une source de lumière extérieure au cerveau est guidée vers le cerveau. Un exemple d'un tel dispositif est décrit dans la demande de brevet FR3010321 précédemment déposée par le demandeur.

Le document US2011/125077 décrit un autre exemple de dispositif pour le traitement par stimulation optique profonde du cerveau.

Il serait toutefois souhaitable de pouvoir améliorer au moins en partie certains aspects des dispositifs connus de stimulation optique profonde du cerveau.

### Résumé

L'invention est définie dans les revendications attenantes. Ainsi, un mode de réalisation prévoit un dispositif comportant : une source lumineuse montée dans un boîtier, le boîtier comportant un hublot transparent en regard de la source lumineuse ; une fibre optique équipée d'un connecteur adapté à coopérer de façon détachable avec le boîtier pour maintenir une face d'entrée de la fibre optique en vis-à-vis de la source lumineuse au travers du hublot ; et un élément d'interface en un matériau élastomère transparent adapté, en position connectée, à être maintenu comprimé entre le hublot et la face d'entrée de la fibre optique.

Selon un mode de réalisation, en regard de sa région de mise en contact avec la face d'entrée de la fibre optique, l'élément d'interface présente une première épaisseur en position déconnectée et une deuxième épaisseur en position connectée, la première épaisseur étant 1,1 à 1,3 fois supérieure à la deuxième épaisseur.

Selon un mode de réalisation, en position déconnectée, la surface de l'élément d'interface destinée à être mise en contact avec la face d'entrée de la fibre optique est bombée.

Selon un mode de réalisation, en position déconnectée, la surface de l'élément d'interface destinée à être mise en contact avec la face d'entrée de la fibre optique présente des nano-structurations ou des micro-structurations.

Selon un mode de réalisation, l'élément d'interface est en silicone ou en polyuréthane.

Selon un mode de réalisation, la source lumineuse est une source laser.

Selon un mode de réalisation, le hublot est en saphir ou en silice.

Selon un mode de réalisation, le boîtier est hermétiquement fermé.

Selon un mode de réalisation, le dispositif comporte en outre un système optique adapté, en position connectée, à focaliser la lumière émise par la source sur la face d'entrée de la fibre optique.

Selon un mode de réalisation, l'élément d'interface est monté solidaire du boîtier ou du connecteur.

Selon un mode de réalisation, le dispositif comporte en outre un module d'alimentation et de commande relié à la source lumineuse par un câble de liaison.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une représentation schématique d'un exemple d'un dispositif implantable pour l'irradiation optique du cerveau ;
la figure 2 est une vue en coupe illustrant plus en détail un exemple d'un dispositif implantable pour l'irradiation optique du cerveau ;
les figures 3A et 3B sont des vues en coupe illustrant un exemple d'un mode de réalisation d'un dispositif implantable pour l'irradiation optique du cerveau ;
la figure 4 est une vue en coupe illustrant une variante de réalisation du dispositif des figures 3A et 3B ;
la figure 5 est une vue en coupe illustrant une autre variante de réalisation du dispositif des figures 3A et 3B ; et
la figure 6 est une vue en coupe illustrant une autre variante de réalisation du dispositif des figures 3A et 3B.

### Description détaillée

De mêmes éléments ont été désignés par de mêmes références aux différentes figures et, de plus, les diverses figures ne sont pas tracées à l'échelle. Par souci de clarté, seuls les éléments qui sont utiles à la compréhension des modes de réalisation décrits ont été représentés et sont détaillés. Sauf précision contraire, les expressions "approximativement", "sensiblement", et "de l'ordre de" signifient à 10 % près, de préférence à 5 % près.

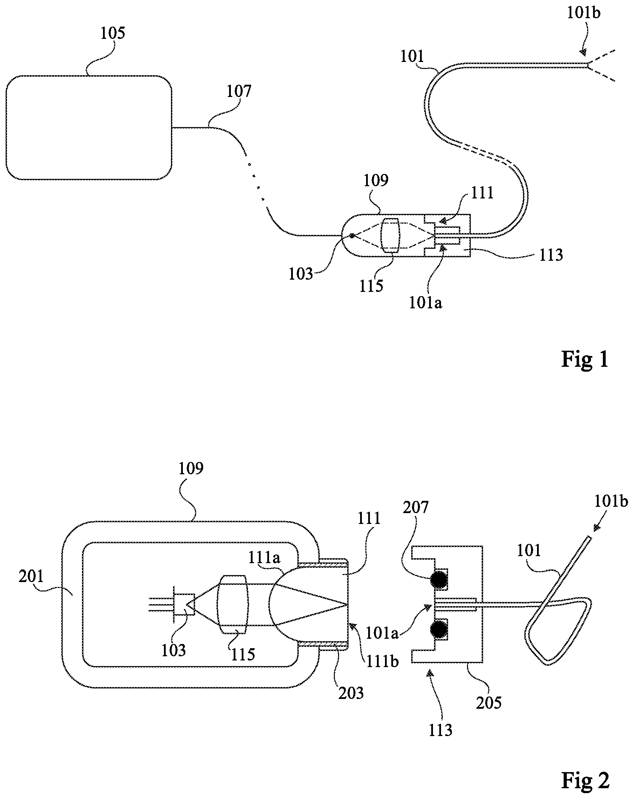

La figure 1 est une représentation schématique d'un exemple d'un dispositif implantable pour l'irradiation optique du cerveau. Ce dispositif comporte une fibre optique 101 dont une première extrémité ou extrémité distale 101b est destinée à être implantée à l'intérieur du cerveau, en vis-à-vis d'une partie du cerveau que l'on souhaite stimuler. Le dispositif de la figure 1 comprend en outre une source lumineuse 103 couplée à une deuxième extrémité ou extrémité proximale 101a de la fibre optique 101. Le dispositif de la figure 1 comprend de plus un module 105 d'alimentation et de commande de la source lumineuse 103, comportant par exemple une batterie électrique et un circuit de contrôle adapté à commander la source lumineuse 103 pour mettre en oeuvre des stimulations souhaitées du cerveau. En pratique, pour minimiser les risques pour le patient, seule la fibre optique 101 est effectivement implantée dans le cerveau du patient, les autres éléments étant maintenus à l'extérieur du cerveau pendant toute la durée du traitement. La source lumineuse 103 et le module d'alimentation et de contrôle 105 peuvent par exemple être implantés dans d'autres parties du corps du patient. A titre d'exemple, la source lumineuse 103 peut être implantée sous le cuir chevelu ou dans la boîte crânienne du patient, et le module d'alimentation et de contrôle 105 peut être implanté au niveau du thorax du patient. Dans ce cas, le dispositif peut comporter un câble de liaison 107 reliant le module 105 à la source lumineuse 103. A titre de variante, la source lumineuse 103 peut être montée solidaire du module d'alimentation et de commande 105.

Pour pouvoir effectuer certaines opérations de réglage ou de maintenance sans avoir à extraire la fibre optique 101 du cerveau du patient, il est important que la source lumineuse 103 soit couplée de manière détachable ou amovible à la fibre optique 101. Pour cela, la source 103 est disposée dans un boîtier de protection 109, ce boîtier comportant un hublot transparent 111 en vis-à-vis de la source lumineuse 103. De plus, le dispositif comprend, du côté de l'extrémité proximale 101a de la fibre optique 101, un connecteur 113 monté solidaire de la fibre optique 101. Le connecteur 113 est adapté à coopérer de façon détachable ou amovible avec le boîtier 109 pour maintenir l'extrémité proximale 101a de la fibre optique en vis-à-vis de la source lumineuse 103 au travers du hublot 111. Dans l'exemple représenté, le dispositif comporte en outre, à l'intérieur du boîtier 109, un système optique 115 adapté à focaliser la lumière émise par la source 103 sur la face d'entrée 101a de la fibre optique 101 en position connectée, c'est-à-dire lorsque le connecteur 113 est connecté au boîtier 109. En position connectée, la lumière émise par la source lumineuse 103 pénètre dans la fibre optique 101 par son extrémité proximale 101a, est guidée jusqu'à l'intérieur du cerveau par la fibre optique 101, puis ressort de la fibre optique 101 par son extrémité distale 101b pour éclairer le cerveau.

Un problème qui se pose est celui du maintien de la propreté à l'interface entre le hublot 111 du boîtier de protection 109 de la source lumineuse 103 et la face d'entrée 101a de la fibre optique 101. Il convient en effet d'éviter que des impuretés ne s'interposent entre le hublot 111 et la face d'entrée 101a de la fibre optique 101, ce qui dégraderait la qualité de la transmission optique. Ce problème se pose tout particulièrement dans le cas de dispositifs implantables, dans lesquels du liquide physiologique pourrait s'interposer entre le hublot 111 et la face d'entrée 101a de la fibre optique 101 (pendant l'acte chirurgical, ou par la suite d'une infiltration).

La figure 2 est une vue en coupe illustrant plus en détail un exemple d'un dispositif du type décrit en relation avec la figure 1. Par souci de simplification, le module d'alimentation et de contrôle 105 et la liaison 107 entre le module 105 et la source lumineuse 103 n'ont pas été représentés sur la figure 2. Seuls la source lumineuse 103, le boîtier de protection 109 et son hublot 111, le système optique 115, le connecteur 113, et la fibre optique 101 ont été représentés sur la figure 2.

La source lumineuse 103 est par exemple une source laser. A titre d'exemple, la source 103 est une source infrarouge, par exemple émettant dans une bande de longueurs d'ondes comprise dans la plage allant de 650 à 1100 nm. La fibre optique 101 peut comprendre une gaine extérieure en un matériau biocompatible, et, du côté de son extrémité distale 101b et/ou du côté de son extrémité proximale 101a, un manchon de protection en un matériau biocompatible transparent. Le boîtier 109 est par exemple réalisé en un ou plusieurs matériaux biocompatibles. A titre d'exemple, le boîtier 109 comprend un corps 201 en un matériau biocompatible opaque, par exemple du titane, le hublot 111 pouvant être réalisé en un matériau biocompatible transparent, par exemple du saphir ou de la silice. Le boîtier 109 est par exemple adapté à isoler de façon hermétique l'ensemble des composants non biocompatibles qu'il contient. A titre d'exemple, le boîtier 109 est fermé sous atmosphère neutre (sans oxygène), par exemple sous argon. Le hublot 111 est par exemple disposé dans une ouverture du corps 201, une brasure hermétique biocompatible 203, par exemple une brasure à l'or, assurant l'étanchéité entre les parois de l'ouverture et le hublot. Les traversées électriques (non représentées) du boîtier 109, permettant de relier électriquement la source lumineuse 103 au module d'alimentation et de commande 105, sont par exemple réalisées en des matériaux biocompatibles, par exemple du platine iridié pour les parties conductrices et de la céramique ou du saphir pour les parties isolantes. Ces traversées électriques peuvent être fermées par brasure hermétique biocompatible, par exemple par brasure à l'or. De même, le connecteur 113 peut être réalisé en un ou plusieurs matériaux biocompatibles.

Le hublot 111 comprend, du côté extérieur du boîtier 109, une face extérieure ou face de sortie 111b, par exemple plane. Lorsque le connecteur 113 est connecté au boîtier 109, la face de sortie 111b du hublot 111 est en contact avec la face d'entrée 101a de la fibre optique 101. Le hublot 111 comprend en outre, du côté intérieur du boîtier (à l'opposé de la face 111b), une face intérieure ou face d'entrée 111a. Dans l'exemple de la figure 2, la face d'entrée 111a du hublot 111 est bombée, de sorte que le hublot 111 se comporte comme une lentille et contribue à la mise en forme du faisceau émis par la source 103. Dans cet exemple, l'ensemble optique comprenant le système optique 115 et le hublot 111 est adapté à focaliser la lumière émise par la source lumineuse 103 sensiblement sur la face de sortie 111b du hublot 111. Le hublot 111 peut comporter une couche antireflet sur sa face intérieure 111a.

Le connecteur 113 comprend un corps 205, par exemple en titane, monté solidaire d'une partie proximale de la fibre optique 101. La face d'entrée 101a de la fibre optique 101 affleure sensiblement au niveau d'une face du corps 205 placée en contact avec la face extérieure 111a du hublot lorsque le connecteur 113 est connecté au boîtier 109. Par souci de simplification, les éléments de blocage du connecteur 113 en position connectée n'ont pas été représentés. Ces éléments peuvent comporter par exemple une bague de serrage à vis, un mécanisme de serrage à baïonnette, ou tout autre mécanisme de blocage adapté.

Pour empêcher que des substances susceptibles de dégrader la qualité de la transmission optique ne puissent s'interposer entre la face de sortie 111b du hublot 111 et la face d'entrée 101a de la fibre optique 101, le dispositif de la figure 2 comprend en outre un joint torique 207 entourant l'extrémité proximale 101a de la fibre optique 101. En position connectée, le joint torique 207 est maintenu comprimé entre le corps 205 du connecteur 113 et la face extérieure 111b du hublot, de façon à empêcher la pénétration des substances indésirables dans la zone de couplage optique entre le hublot 111 et la fibre optique 101.

Un inconvénient du dispositif de la figure 2 réside dans les dimensions relativement importantes du connecteur 113, liées notamment à la présence du joint torique 207 entourant la face d'entrée 101a de la fibre optique 101. Il est en outre difficile de garantir un bon contact entre la fibre et le hublot. De plus, des problèmes de réflexion de la lumière, notamment directement sur le laser lui-même, peuvent se poser en cas de mauvaise adaptation des indices. Ceci peut entrainer une instabilité du laser et provoquer une défaillance de celui-ci.

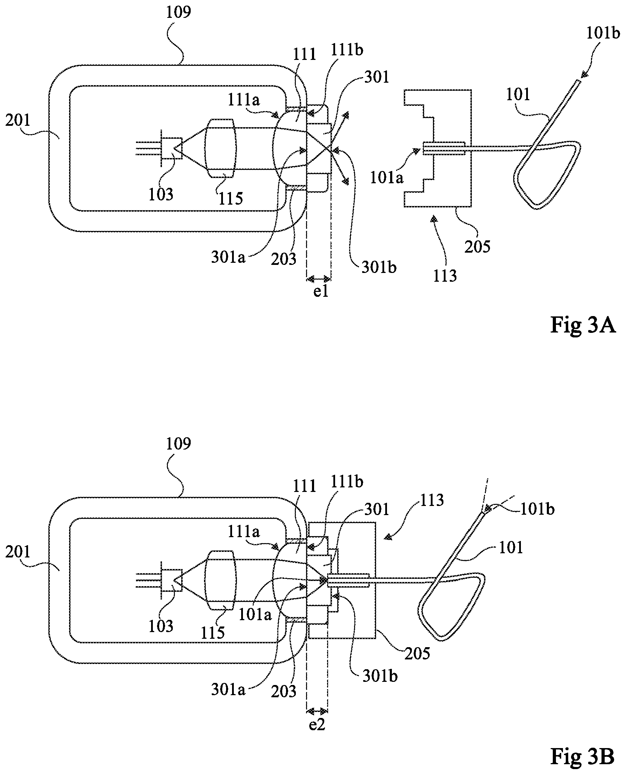

Les figures 3A et 3B sont des vues en coupe illustrant un exemple d'un mode de réalisation d'un dispositif implantable pour l'irradiation optique du cerveau. Le dispositif des figures 3A et 3B comprend des éléments communs avec le dispositif des figures 1 et 2. Ces éléments ne seront pas décrits à nouveau en détail ci-après. Dans la suite, seules les différences avec le dispositif des figures 1 et 2 seront mises en exergue.

Comme dans l'exemple de la figure 2, le dispositif des figures 3A et 3B comprend une fibre optique 101, une source de lumière 103, un boîtier 109 de protection de la source lumineuse 103 muni d'un hublot transparent 111, un système optique 115 de mise en forme de la lumière émise par la source 103, et un connecteur 113 adapté à connecter la fibre optique 101 au boîtier 109 pour réaliser un couplage optique entre la source lumineuse 103 et la fibre optique 101 au travers du hublot 111.

La figure 3A représente le dispositif en position déconnectée, c'est-à-dire lorsque le connecteur 113 est déconnecté du boîtier 109, la fibre optique 101 n'étant alors pas couplée à la source lumineuse 103. La figure 3B représente le dispositif en position connectée, c'est-à-dire lorsque le connecteur 113 est connecté au boîtier 109, la fibre optique 101 étant alors couplée optiquement à la source lumineuse 103. De même que sur la figure 2, les éléments de blocage en position connectée et de déblocage du connecteur 113 n'ont pas été représentés sur les figures 3A et 3B.

Le dispositif des figures 3A et 3B diffère du dispositif de la figure 2 en ce qu'il ne comprend pas de joint torique autour de la face d'entrée 101a de la fibre optique 101, entre le corps 205 du connecteur 113 et le boîtier 109.

Le dispositif des figures 3A et 3B comprend en revanche un élément d'interface 301 en un matériau élastomère transparent adapté à être maintenu pressé ou comprimé entre la face de sortie 111b du hublot 111 et la face d'entrée 101a de la fibre optique 101 lorsque le connecteur 113 est connecté au boîtier 109. L'élément d'interface 301 est par exemple une plaque ou un disque. A titre d'exemple, l'élément d'interface 301 est en matériau présentant un module de Young compris entre 1 et 100 MPa. L'élément d'interface 301 est par exemple en un matériau biocompatible. A titre d'exemple, l'élément d'interface 301 est en silicone ou en polyuréthane. L'élément d'interface 301 comprend une première face ou face d'entrée 301a, en contact avec la face de sortie 111b du hublot 111 lorsque le connecteur 113 est connecté au boîtier 109. L'élément d'interface 301 comprend en outre une deuxième face ou face de sortie 301b opposée à la face 301a, en contact avec la face d'entrée 101a de la fibre optique 101 lorsque le connecteur 113 est connecté au boîtier 109. Les faces 301a et 301b de l'élément 301 sont par exemple sensiblement planes et parallèles entre elles. L'élément d'interface 301 est par exemple solidaire du boîtier 109 à l'état déconnecté. A titre d'exemple, l'élément d'interface 301 peut être coulé en place en regard du hublot 111 dans un logement du boîtier 109 prévu à cet effet. A titre de variante, l'élément 301 peut être solidaire du connecteur 113 à l'état déconnecté. A titre de variante, l'élément 301 peut être une pièce amovible indépendante, c'est-à-dire non fixée au connecteur 113 ou au boîtier 109 à l'état déconnecté.

En position déconnectée (figure 3A), l'élément 301 présente une première épaisseur (ou distance entre ses faces 301a et 301b) e1 au niveau de sa région de couplage avec la fibre optique 101, c'est-à-dire en regard de la portion de sa face 301b destinée à être mise en contact avec la face d'entrée 101a de la fibre optique 101 lors de la connexion du connecteur 113 au boîtier. A titre d'exemple, l'épaisseur e1 est comprise entre 0,5 et 2 mm.

En position déconnectée (figure 3B), l'épaisseur e2 de l'élément 301 en regard de la face d'entrée 101a de la fibre optique 101 est inférieure à l'épaisseur e1. A titre d'exemple, l'épaisseur e1 de l'élément 301 en position déconnectée est comprise entre 1,1 et 1,3 fois l'épaisseur e2 de l'élément 301 en position connectée. En pratique, le connecteur 113 et/ou le boitier 109 peuvent définir un évent ou logement adapté à contenir le surplus de matière en périphérie de la zone de couplage avec la fibre optique 101, dans le cas où l'élément 301 est en un matériau non compressible ou faiblement compressible.

Dans le dispositif des figures 3A et 3B, l'ensemble optique comprenant le système optique 115, le hublot 111 et l'élément d'interface 301 est adapté à focaliser la lumière produite par la source 103 à l'extérieur du boîtier 109, à une distance de la face de sortie 111b du hublot 111 sensiblement égale à l'épaisseur e2 de l'élément d'interface 301 en position connectée. Ainsi, en position connectée, la lumière produite par la source 103 est focalisée sensiblement sur la face d'entrée 101a de la fibre optique 101, et conduite par la fibre optique 101 jusqu'à sa face de sortie 101b. On notera que bien que le hublot 111 ait été représenté avec une face d'entrée 111b bombée dans l'exemple des figures 3A et 3B, les modes de réalisation décrits ne se limitent pas à ce cas particulier. A titre de variante, le hublot 111 peut avoir une face d'entrée 111b plane sensiblement parallèle à sa face de sortie 111a, ou une face d'entrée 111b concave.

Du fait de ses propriétés élastiques, et de par son maintien en compression entre le hublot 111 et la fibre optique 101, l'élément d'interface 301 forme un joint empêchant, en position connectée, que des substances indésirables s'interposent entre la face de sortie 111b du hublot 111 et la face d'entrée 101a de la fibre optique 101. Il n'est alors pas nécessaire de prévoir un joint en anneau autour de la face d'entrée 101a de la fibre optique 101, ce qui permet de réduire les dimensions du connecteur 113 par rapport à un système du type décrit en relation avec la figure 2.

Un autre avantage du dispositif des figures 3A et 3B est que l'élément d'interface 301b permet de réduire les pertes par réflexion et/ou les phénomènes parasites de rétrodiffusion de la lumière sur la source 103, particulièrement problématiques dans le cas d'une source laser. A titre d'exemple, l'élément d'interface 301 est en un matériau d'indice optique sensiblement égal à celui du matériau de la face d'entrée 101a de la fibre optique 101, ou en un matériau d'indice optique intermédiaire entre celui du matériau du hublot 111 et celui du matériau de la face d'entrée 101a de la fibre optique 101. Les réflexions parasites de la lumière sur la face d'entrée 101a de la fibre optique sont ainsi réduites par rapport à un dispositif du type décrit en relation avec la figure 2. Dans le dispositif des figures 3A et 3B, des réflexions parasites peuvent se produire sur la face d'entrée 301a de l'élément d'interface 301. Ces réflexions sont toutefois moins gênantes dans la mesure où elles se produisent en dehors du plan focal du système optique.

On notera en outre que dans le dispositif des figures 3A et 3B, du fait des propriétés élastiques de l'élément d'interface 301, la marge de tolérance de positionnement de la face d'entrée 101a de la fibre optique 101 dans l'axe de la source 103 est supérieure à celle d'un système du type décrit en relation avec la figure 2, dans lequel le hublot rigide est mis en contact directement avec la face d'entrée 101a de la fibre optique 101 (un mécanisme à ressort doit alors en pratique être prévu pour maintenir la face d'entrée 101a de la fibre optique 101 appuyée contre la face de sortie 111b du hublot 111, avec une force de maintien choisie indépendamment de la force d'écrasement du joint torique 207). Ceci permet de simplifier le connecteur 113 par rapport à un dispositif du type décrit en relation avec la figure 2.

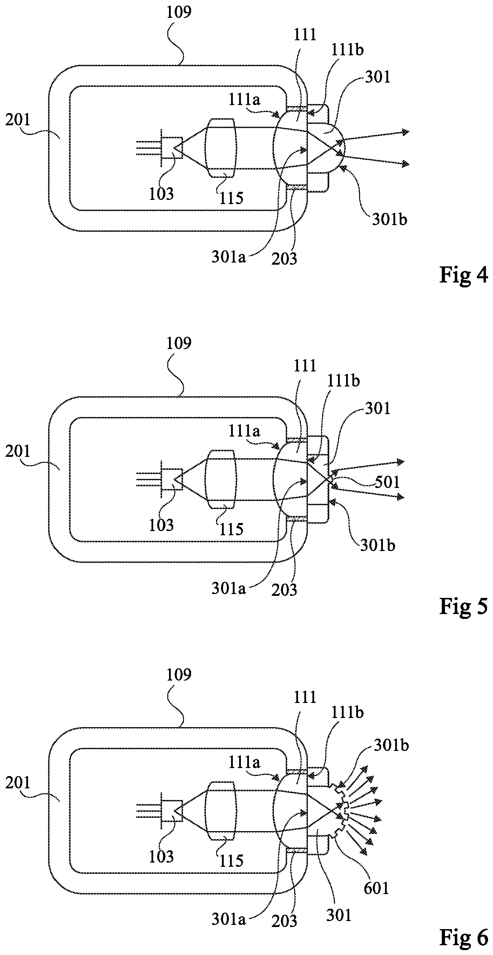

La figure 4 est une vue en coupe illustrant une variante de réalisation du dispositif des figures 3A et 3B. Le dispositif de la figure 4 diffère du dispositif des figures 3A et 3B essentiellement en ce que, dans l'exemple de la figure 4, la face de sortie 301b de l'élément d'interface 301 n'est pas plane, mais présente une surface bombée ou convexe, ce qui permet de limiter les risques de piégeage d'une éventuelle bulle d'air entre la face de sortie 301b de l'élément 301 et la face d'entrée 101a de la fibre optique 101 lors de la connexion du connecteur 113 au boîtier 109. En position connectée, du fait de l'écrasement de l'élément 301, la surface de l'élément 301 en contact avec la face d'entrée 101a de la fibre optique 101 redevient sensiblement plane et parallèle à la face d'entrée 301a de l'élément d'interface 301.

La figure 5 est une vue en coupe illustrant une autre variante de réalisation du dispositif des figures 3A et 3B. Le dispositif de la figure 5 diffère du dispositif de la figure 4 essentiellement en ce que, dans l'exemple de la figure 5, la face de sortie 301b de l'élément d'interface 301 n'est pas bombée sur toute sa surface, mais présente une excroissance bombée 501 dans une partie centrale destinée à être mise en contact avec la face d'entrée 101a de la fibre optique 101. La partie périphérique de la face de sortie 301b de l'élément d'interface 301 est quant à elle sensiblement parallèle à la face d'entrée 301a de l'élément 301. Comme dans l'exemple de la figure 4, la prévision d'une partie bombée sur la surface 301b de l'élément 301 permet de limiter les risques de piégeage d'une éventuelle bulle d'air entre la face 301b de l'élément 301 et la face d'entrée 101a de la fibre optique 101 lors de la connexion du connecteur 113 au boîtier 109. En position connectée, du fait de l'écrasement de l'élément 301, la surface de l'élément 301 en contact avec la face d'entrée 101a de la fibre optique 101 redevient sensiblement plane et parallèle à la face d'entrée 301a de l'élément d'interface 301.

Dans une autre variante de réalisation (non représentée), la face d'entrée 101a de la fibre optique 101 n'est pas orthogonale à l'axe principal de propagation de la lumière, mais présente une inclinaison, par exemple de l'ordre de 8 à 12 degrés, par rapport à cet axe, de façon à rejeter le faisceau en dehors de l'angle de couplage du laser.

La figure 6 est une vue en coupe illustrant une autre variante de réalisation du dispositif des figures 3A et 3B. Le dispositif de la figure 6 diffère du dispositif de la figure 4 essentiellement en ce que, dans l'exemple de la figure 6, la face de sortie 301b de l'élément d'interface 301 n'est pas lisse, mais présente des nano-structurations ou des micro-structurations 601, par exemple des structurations de dimensions comprises entre 200 nm et 200 µm, sur tout ou partie de sa surface. En position déconnectée, les structurations 601 diffractent le faisceau lumineux, ce qui réduit les risques d'éblouissement ou de brulure pour un opérateur manipulant le dispositif, notamment lorsque la source 103 est une source laser. En position connectée, du fait de l'écrasement de l'élément 301, la surface de l'élément 301 en contact avec la face d'entrée 101a de la fibre optique 101 redevient lisse. Bien que la figure 6 ait été représentée avec un élément d'interface 301 présentant une face de sortie 301b de forme générale bombée (similaire à celle du dispositif de la figure 4), la variante de la figure 6 peut être mise en oeuvre quelle que soit la forme générale de la face de sortie 301b de l'élément d'interface 301.

Des modes de réalisation particuliers ont été décrits. Diverses variantes et modifications apparaîtront à l'homme de l'art. En particulier, les modes de réalisation décrits ne se limitent pas à l'exemple particulier d'application à un dispositif médical implantable décrit ci-dessus. Plus généralement, la solution proposée peut avoir des applications dans d'autres domaines dans lesquels on souhaite coupler une source lumineuse à une fibre optique en protégeant contre des impuretés l'interface de couplage optique entre la source lumineuse et la fibre optique. Le choix des matériaux peut notamment être adapté en conséquence.

## Revendications

1. Dispositif comportant :
une source lumineuse (103) montée dans un boîtier (109), le boîtier (109) comportant un hublot transparent (111) en regard de la source lumineuse (103) ;
une fibre optique (101) équipée d'un connecteur (113) adapté à coopérer de façon détachable avec le boîtier (109) pour maintenir une face d'entrée (101a) de la fibre optique (101) en vis-à-vis de la source lumineuse (103) au travers du hublot (111) ; et
un élément d'interface (301) en un matériau élastomère transparent adapté, en position connectée, à être maintenu comprimé entre le hublot (111) et la face d'entrée (101a) de la fibre optique (101).

2. Dispositif selon la revendication 1, dans lequel en regard de sa région de mise en contact avec la face d'entrée (101a) de la fibre optique (101), l'élément d'interface (301) présente une première épaisseur (e1) en position déconnectée et une deuxième épaisseur (e2) en position connectée, la première épaisseur (e1) étant 1,1 à 1,3 fois supérieure à la deuxième épaisseur (e2).

3. Dispositif selon la revendication 1 ou 2, dans lequel, en position déconnectée, la surface de l'élément d'interface (301) destinée à être mise en contact avec la face d'entrée (101a) de la fibre optique (101) est bombée.

4. Dispositif selon l'une quelconque des revendications 1 à 3, dans lequel, en position déconnectée, la surface de l'élément d'interface (301) destinée à être mise en contact avec la face d'entrée (101a) de la fibre optique (101) présente des nano-structurations ou des micro-structurations (601).

5. Dispositif selon l'une quelconque des revendications 1 à 4, dans lequel l'élément d'interface (301) est en silicone ou en polyuréthane.

6. Dispositif selon l'une quelconque des revendications 1 à 5, dans lequel la source lumineuse (103) est une source laser.

7. Dispositif selon l'une quelconque des revendications 1 à 6, dans lequel le hublot (111) est en saphir ou en silice.

8. Dispositif selon l'une quelconque des revendications 1 à 7, dans lequel le boîtier (109) est hermétiquement fermé.

9. Dispositif selon l'une quelconque des revendications 1 à 8, comportant en outre un système optique (115) adapté, en position connectée, à focaliser la lumière émise par la source (103) sur la face d'entrée (101a) de la fibre optique (101).

10. Dispositif selon l'une quelconque des revendications 1 à 9, dans lequel l'élément d'interface (301) est monté solidaire du boîtier (109) ou du connecteur (113).

11. Dispositif selon l'une quelconque des revendications 1 à 10, comportant en outre un module d'alimentation et de commande (105) relié à la source lumineuse (103) par un câble de liaison (107) .

## Patentansprüche

1. Eine Vorrichtung, die Folgendes aufweist:
eine Lichtquelle (103), die in einer Verpackung (109) montiert ist, wobei die Verpackung (109) ein transparentes Fenster (111) gegenüber der Lichtquelle (103) umfasst;
eine optische Faser (101), die mit einem Verbinder (113) ausgestattet ist, der in der Lage ist, lösbar mit dem Gehäuse (109) in Eingriff zu kommen, um eine Eingangsfläche (101a) der optischen Faser (101) gegenüber der Lichtquelle (103) über das Fenster (111) zu halten; und
ein Schnittstellenelement (301) aus einem transparenten elastomeren Material, das in verbundener Position zwischen dem Fenster (111) und der Eingangsfläche (101a) der optischen Faser (101) komprimiert gehalten werden kann.

2. Vorrichtung nach Anspruch 1, wobei das Schnittstellenelement (301) gegenüber dem Bereich, in dem es in Kontakt mit der Eingangsfläche (101a) der Glasfaser (101) gebracht wird, eine erste Dicke (e1) in getrennter Position und eine zweite Dicke (e2) in verbundener Position aufweist, wobei die erste Dicke (e1) 1,1 bis 1,3 mal größer als die zweite Dicke (e2) ist.

3. Vorrichtung nach Anspruch 1 oder 2, wobei in getrennter Position die Oberfläche des Schnittstellenelements (301), das dazu bestimmt ist, mit der Eingangsoberfläche (101a) der optischen Faser (101) in Kontakt gebracht zu werden, gewölbt ist.

4. Vorrichtung nach einem der Ansprüche 1 bis 3, wobei die Oberfläche des Schnittstellenelements (301), die dazu bestimmt ist, mit der Eingangsoberfläche (101a) der optischen Faser (101) in Kontakt gebracht zu werden, in getrennter Position Nanostrukturen oder Mikrostrukturen (601) aufweist.

5. Vorrichtung nach einem der Ansprüche 1 bis 4, wobei das Schnittstellenelement (301) aus Silikon oder Polyurethan hergestellt ist.

6. Vorrichtung nach einem der Ansprüche 1 bis 5, wobei die Lichtquelle (103) eine Laserquelle ist.

7. Vorrichtung nach einem der Ansprüche 1 bis 6, wobei das Fenster (111) aus Saphir oder Siliziumdioxid besteht.

8. Vorrichtung nach einem der Ansprüche 1 bis 7, wobei die Verpackung (109) hermetisch verschlossen ist.

9. Vorrichtung nach einem der Ansprüche 1 bis 8, ferner umfassend ein optisches System (115), das in verbundener Position in der Lage ist, das von der Quelle (103) emittierte Licht auf die Eingangsfläche (101a) der optischen Faser (101) zu fokussieren.

10. Vorrichtung nach einem der Ansprüche 1 bis 9, wobei das Schnittstellenelement (301) starr an dem Gehäuse (109) oder an dem Verbinder (113) befestigt ist.

11. Vorrichtung nach einem der Ansprüche 1 bis 10, ferner umfassend eine Stromversorgungs- und Steuereinheit (105), die über ein Verbindungskabel (107) mit der Lichtquelle (103) gekoppelt ist.

## Claims

1. A device comprising:
a light source (103) assembled in a package (109), the package (109) comprising a transparent window (111) opposite the light source (103);
an optical fiber (101) fitted with a connector (113) capable of detachably engaging with the package (109) to maintain an input surface (101a) of the optical fiber (101) opposite the light source (103) via the window (111); and
an interface element (301) made of a transparent elastomeric material capable, in connected position, of being maintained compressed between the window (111) and the input surface (101a) of the optical fiber (101).

2. The device of claim 1, wherein, opposite the region where it is placed in contact with the input surface (101a) of the optical fiber (101), the interface element (301) has a first thickness (e1) in disconnected position and a second thickness (e2) in connected position, the first thickness (e1) being from 1.1 to 1.3 times greater than the second thickness (e2).

3. The device of claim 1 or 2, wherein, in disconnected position, the surface of the interface element (301) intended to be placed in contact with the input surface (101a) of the optical fiber (101) is bulged.

4. The device of any of claims 1 to 3, wherein, in disconnected position, the surface of the interface element (301) intended to be placed in contact with the input surface (101a) of the optical fiber (101) has nanostructures or microstructures (601) .

5. The device of any of claims 1 to 4, wherein the interface element (301) is made of silicone or of polyurethane.

6. The device of any of claims 1 to 5, wherein the light source (103) is a laser source.

7. The device of any of claims 1 to 6, wherein the window (111) is made of sapphire or of silica.

8. The device of any of claims 1 to 7, wherein the package (109) is hermetically closed.

9. The device of any of claims 1 to 8, further comprising an optical system (115) capable, in connected position, of focusing the light emitted by the source (103) onto the input surface (101a) of the optical fiber (101).

10. The device of any of claims 1 to 9, wherein the interface element (301) is rigidly assembled to the package (109) or to the connector (113).

11. The device of any of claims 1 to 10, further comprising a power supply and control unit (105) coupled to the light source (103) by a connecting cable (107).
